# EUROPÄISCHE PATENTSCHRIFT

(11) **EP 3 717 076 B1**
(45) Veröffentlichungstag und Bekanntmachung des Hinweises auf die Patenterteilung: **22.07.2026**
(21) Anmeldenummer: 18812081.0
(22) Anmeldetag: 20.11.2018
(51) Int. Cl.: A61Q 19/00, A61K 8/92

(54) **BRUCHFESTER, MINERALÖLFREIER LIPPENSTIFT**
BREAK-PROOF, MINERAL OIL-FREE LIPSTICK
ROUGE À LÈVRES SANS HUILE MINÉRALE RÉSISTANT À LA RUPTURE

(30) Priorität: 01.12.2017 DE 102017221672
(43) Veröffentlichungstag der Anmeldung: 07.10.2020
(73) Patentinhaber: Beiersdorf AG, 22529 Hamburg (DE)
(72) Erfinder: SCHECKER, Franziska, D-21079 Hamburg (DE); ZEWUHN, Merle, 25335 Elmshorn (DE)
(74) Vertreter: Beiersdorf AG
(86) Internationale Anmeldenummer: PCT/EP2018/081884
(87) Internationale Veröffentlichungsnummer: WO 2019/105799

(56) Entgegenhaltungen:
- WO-A1-2016/007519
- WO-A1-2018/162288
- WO-A2-2012/158627
- DE-A1- 102012 207 989
- DE-A1- 102014 204 454
- DE-A1- 102014 204 477
- US-A1- 2017 258 706
- DATABASE GNPD [online] MINTEL; 31 January 2017 (2017-01-31), ANONYMOUS: "Amuse Bouche Lipstick", XP055545252, retrieved from https://www.gnpd.com/sinatra/recordpage/4578683/ Database accession no. 4578683
- DATABASE GNPD [online] MINTEL; 8 November 2017 (2017-11-08), ANONYMOUS: "Hugs & Kisses", XP055545253, retrieved from https://www.gnpd.com/sinatra/recordpage/5224749/ Database accession no. 5224749
- DATABASE GNPD [online] MINTEL; 18 September 2017 (2017-09-18), ANONYMOUS: "Moisturising Lip Balm Stick with Olive Oil", XP055545155, retrieved from https://www.gnpd.com/sinatra/recordpage/5104825/ Database accession no. 5104825
- DATABASE GNPD [online] MINTEL; 3 January 2017 (2017-01-03), ANONYMOUS: "Milano Red Lipstick", XP055545319, retrieved from https://www.gnpd.com/sinatra/recordpage/4512059/ Database accession no. 4512059
- DATABASE GNPD [online] MINTEL; 18 September 2017 (2017-09-18), ANONYMOUS: "Moisturising Lip Balm Stick with Olive Oil", XP055545155, retrieved from www.gnpd.com Database accession no. 5104825
- DATABASE GNPD [online] MINTEL; 31 January 2017 (2017-01-31), ANONYMOUS: "Amuse Bouche Lipstick", XP055545252, retrieved from www.gnpd.com Database accession no. 4578683
- DATABASE GNPD [online] MINTEL; 8 November 2017 (2017-11-08), ANONYMOUS: "Hugs & Kisses", XP055545253, retrieved from www.gnpd.com Database accession no. 5224749
- DATABASE GNPD [online] MINTEL; 3 January 2017 (2017-01-03), ANONYMOUS: "Milano Red Lipstick", XP055545319, retrieved from www.gnpd.com Database accession no. 4512059

## Beschreibung

Die vorliegende Erfindung betrifft eine Lippenpflegezubereitung, insbesondere einen Lippenstift, sowie das Verfahren zur Herstellung eines Lippenstiftes.

Der Wunsch, schön und attraktiv auszusehen, ist seit Tausenden von Jahren in den Menschen verwurzelt. Auch wenn das Schönheitsideal im Laufe der Zeit Wandlungen erfahren hat, so ist das Streben nach einem makellosen Äußeren, immer das Ziel der Menschen gewesen, da ein sympathisches Erscheinungsbild ihr Selbstwertgefühl und die Anziehungskraft auf ihre Mitmenschen erhöht.

Der Begriff der dekorativen Kosmetik leitet vom lateinischen "decoratio" - das Hervorheben des Schönen - ab. Meist werden dabei mit Hilfe von Farbstoffen einzelne Körperpartien, insbesondere im Gesicht, hervorgehoben und farbliche Uneinheitlichkeiten abgemildert.

Neben Gesichtspudern und Rouge als pulverförmigen Kosmetika sowie stiftförmigen Zubereitungen werden hierzu cremeförmige Präparate, wie Tagescremes und Creme-Make-up auf Emulsionsbasis verwendet.

Zu den am meisten verwendeten dekorativen Kosmetika gehört der Lippenstift. Mehr als 50 % der Frauen in Deutschland benutzen ihn. Meist dient er neben der farblichen Betonung der Lippen auch zur Lippenpflege.

Lippenstifte bestehen in der Regel aus einer Wachsmatrix, in die in geringer Konzentration flüssige und halbfeste Öle sowie Pigmente und Füllstoffe eingearbeitet sind. Sie liegen in fester Stiftform vor.

Nachteilig am Stande der Technik ist der Umstand, dass Lippenpflegeprodukte wie Lippencremes, Lippenbutter etc. und insbesondere die Lippenstifte des Standes der Technik häufig Mineralöle und Paraffinwachse enthalten, die bei den Verbrauchern zunehmend unbeliebt sind. Neben einem generellen Wunsch nach Produkten aus nachwachsenden Rohstoffen, spielt dabei auch die Sorge vor gesundheitsgefährdenden Verunreinigungen in den auf Rohölbasis hergestellte Rohstoffen enthalten sein könnten eine Rolle. Ob diese Sorge tatsächlich berechtigt ist, kann im Rahmen der vorliegenden Offenbarung dahingestellt bleiben. Tatsache ist, dass bei vielen Verbrauchern der Wunsch nach "mineralölfreien" Produkten besteht.

Für die Herstellung insbesondere von Lippenstiften hat der Verzicht auf Mineralöle und Paraffinwachse jedoch gravierende Nachteile. Bei der Herstellung kommt es beim Abkühlungsvorgang der geschmolzenen Stiftmasse leicht zu optischen Mängeln, Rissen und Brüchen in der Stiftmasse, wenn das Temperaturprogramm für diesen Abkühlungsprozess nicht exakt eingehalten wird, beispielsweise wenn zu schnell und mit einer zu hohen Temperaturdifferenz zwischen Schmelze und Kühlung gearbeitet wird. Darüber hinaus gerät die Stiftmasse leicht zu hart, wodurch es beim Entformen (Abfüllen) in die Stifthülsen zu Brüchen in der festen Stiftmasse kommt, oder zu weich, so dass beim Entformen der Stiftmasse Reste in der Stiftform (Gießkranz) zurückbleiben, die in einem aufwendigen Reinigungsprozess vor dem nächsten Befüllen erst wieder gesäubert werden müssen. Die Qualitätsschwankungen der Ersatzstoffe natürlichen Ursprungs tun ihr übriges, um diese negativen Effekte zu verstärken. Insgesamt führt der Austausch von Mineralölprodukten zu einem signifikanten Anstieg von unverkäuflicher Ausschusswahre.

Es war daher die Aufgabe der vorliegenden Erfindung, die Nachteile des Standes der Technik zu vermeiden und ein Lippenpflegeprodukt zu entwickeln, das frei ist von Mineralölprodukten und sich gleichzeitig einfacher und mit geringerem Ausschuss sowie höherer Qualität herstellen lässt.

Insbesondere mineralölfreie Lippenpflegeprodukte (insbesondere Lippenstifte) des Standes der Technik haben darüber hinaus den Nachteil, dass sie nicht besonders lagerstabil sind, sondern relativ stark temperatur- und lichtanfällig sind, d.h. bei Temperaturschwankungen oder Licht relativ leicht inhomogen und brüchig/rissig werden. Mineralölfreie Stifte des Standes der Technik haben darüber hinaus den Nachteil, dass sie im Vergleich zu mineralölhaltigen Stiften auf der Lippe ein schlechteres Gleitvermögen zeigen. Diese Stifte fühlen sich wachsartig/klebrig/unangenehm an.

Es war daher die Aufgabe der vorliegenden Erfindung auch diese Mängel des Standes der Technik zu reduzieren.

Überraschend gelöst werden die Aufgaben durch ein Lippenpflegeprodukt gemäß Anspruch 1. Die Lippenpflegezubereitung, d.h. das Lippenpflegeprodukt wird im Rahmen der vorliegenden Offenbarung auch als Zubereitung bezeichnet.

Zwar kennt der Stand der Technik die Datenbank-Einträge in der GNPD-Datenbank Mintel "Moisturising Lip Balm stick with Olive Oil" (Eintragungsnummer:5104825), "Amuse Bouche Lipstick" (Eintragungsnummer 4578683), "Hughs & Kisses" (Eintragungsnummer: 5224749), "Milano red Lipstick" (Eintragungsnummer: 4512059), sowie die Veröffentlichungen DE 10 2014 204477 A1, DE 10 2014 204454 A1, US 2017/258706 A1, WO2016/007519 A1, DE 10 2012 207989 A1, und WO 2012/158627 A1, doch konnten diese Schriften nicht den Weg zur vorliegenden Erfindung weisen.

Vorteilhafte Ausführungsformen der vorliegenden Erfindung sind dadurch gekennzeichnet, dass die Zubereitung das Rizinuswachs in einer Konzentration von 2 bis 10 Gewichts-%, bezogen auf das Gesamtgewicht der Zubereitung, enthält, wobei ein Konzentrationsbereich von 3 bis 4 Gewichts-%, bezogen auf das Gesamtgewicht der Zubereitung erfindungsgemäß bevorzugt ist. Oberhalb von 10 Gewichts-% wird der Stift hingegen zu wachsartig-weich und instabil. Außerdem ist es erfindungsgemäß vorteilhaft, wenn die Zubereitung das Sonnenblumenwachs in einer Konzentration von 2 bis 10 Gewichts-%, bezogen auf das Gesamtgewicht der Zubereitung, enthält. Hier liegt die erfindungsgemäß bevorzugte Einsatzkonzentration bei 3 bis 5 Gewichts-%, bezogen auf das Gesamtgewicht der Zubereitung. Oberhalb von 10 Gewichts-% hingegen wird bei Lippenstiften die Stiftmasse so hart, dass sie sich nicht mehr vernünftig auf der Haut applizieren lässt. Die erfindungsgemäß Ausführungsformen der vorliegenden Erfindung sind darüber hinaus dadurch gekennzeichnet, dass die Zubereitung das Sonnenblumenwachs und das Rizinuswachs in einem Gewichtsverhältnis von 1:1 bis 4:1 enthält.

Ferner sind die erfindungsgemäßen Lippenpflegeprodukte dadurch gekennzeichnet, dass die Zubereitung das Bienenwachs in einer Konzentration von 5 bis 20 Gewichts-% und bevorzugt von 10 bis 15 Gewichts-%, bezogen auf das Gesamtgewicht der Zubereitung, enthält.

Neben den Wachskomponenten enthält ein erfindungsgemäßes Lippenpflegeprodukt vorteilhaft 30 bis 60 Gewichts-% an Ölen wie Guerbet-Alkohole und/oder Triglyceride. Erfindungsgemäß bevorzugt werden diese Öle gewählt aus der Gruppe der Verbindungen Octyldodecanol, Rizinusöl (INCI: Ricinus Communis Seed Oil), Kokosglyceriden (INCI: Coco Glycerides), Jojobaöl (INCI: Simmondsia Chinensis Seed Oil).

Ferner sind erfindungsgemäß vorteilhafte Lippenpflegeprodukte dadurch gekennzeichnet, dass die Zubereitung Cetylpalmitat, Sheabutter und/oder Cetearylalkohol enthält.

Für Sheabutter beträgt die erfindungsgemäß vorteilhafte Einsatzkonzentration von 0,5 bis 6 Gewichts-%, bezogen auf das Gesamtgesicht der Zubereitung.

Für Cetylpalmitat beträgt die erfindungsgemäß vorteilhafte Einsatzkonzentration von 2 bis 10 Gewichts-%, bezogen auf das Gesamtgesicht der Zubereitung.

Für Cetearylalkohol beträgt die erfindungsgemäß vorteilhafte Einsatzkonzentration von 5 bis 15 Gewichts-%, bezogen auf das Gesamtgesicht der Zubereitung.

Außerdem ist es erfindungsgemäß vorteilhaft, wenn die Lippenpflege-Zubereitung Butylhydroxytoluol (BHT) enthält. Die erfindungsgemäße Einsatzkonzentration für diesen Stoff beträgt von 0,01 bis 0,05 Gewichts-%, bezogen auf das Gesamtgewicht der Zubereitung.

Die erfindungsgemäßen Lippenpflegeprodukte können bis zu 2 Gewichts-%, bezogen auf das Gesamtgewicht der Zubereitung, an Wasser und/oder hydrophilen Bestandteilen wie Glycerin, Panthenol etc. enthalten.

Für bestimmte Einsatzzwecke kann es erfindungsgemäß von Vorteil sein, wenn die Zubereitung UV-Filter, Farbstoffe, Parfümstoffe und/oder Aromastoffe enthält.

Enthält das erfindungsgemäße Lippenpflegeprodukt Farbstoff(e), so ist es erfindungsgemäß vorteilhaft, wenn der oder die Farbstoffe gewählt werden aus den Verbindungen mit den Color-Indices CI77891, CI 77492, CI 15850.

Enthält die erfindungsgemäße Zubereitung UV-Filter, so ist es erfindungsgemäß vorteilhaft, wenn die UV-Filter gewählt werden aus der Gruppe der Verbindungen Octocrylen, Homosalat, Ethylhexylsalicylat, Ethylhexylmethoxycinnamat und Butyl Methoxydibenzoylmethan.

Nicht zuletzt sind die erfindungsgemäß vorteilhaften Ausführungsformen der vorliegenden Erfindung dadurch gekennzeichnet, dass die Zubereitung Bis-Diglyceryl Polyacyladipate-2 enthält. Diese Verbindung kann erfindungsgemäß vorteilhaft in einer Konzentration von 5 bis 15 Gewichts-%, bezogen auf das Gesamtgewicht der Zubereitung, eingesetzt werden.

Erfindungsgemäß bevorzugt liegt die erfindungsgemäße Zubereitung, d.h. das erfindungsgemäße Lippenpflegeprodukt in Form eines Lippenstiftes vor. Dies bedeutet, dass es sich bei dem Lippenpflegeprodukt erfindungsgemäß bevorzugt um einen Lippenstift handelt.

Erfindungsgemäß ist auch das Verfahren zur Herstellung dieses Lippenstiftes, welches dadurch gekennzeichnet ist, dass
zuerst die Rezepturbestandteile in einem Heizkessel aufgeschmolzen und homogen vermischt werden,
die Zubereitung anschließend bei einer Temperatur von 5-15 °C über ihrem Erstarrungspunkt in einen Gießkranz gegossen wird,
dann mit einer Kühltemperatur von +6 °C bis +25 °C abgekühlt wird
und die erstarrte lippenstiftförmige Masse aus dem Gießkranz herausgedrückt und in einen Lippenstiftbehälter eingebracht wird, wobei die Zubereitung das Sonnenblumenwachs und das Rizinuswachs in einem Gewichtsverhältnis von 1:1 bis 4:1 enthält,
sowie
ein Lippenstift, der nach diesem Verfahren hergestellt wurde.

### Beispiel

Das nachfolgende Beispiel soll die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen. Die Zusammensetzung gemäß der Erfindung enthält das Sonnenblumenwachs und das Rizinuswachs in einem Gewichtsverhältnis von 1:1 bis 4:1.

| | | |
|---|---|---|
| Phase A | Rizinuswachs | 4% |
| | Sonnenblumenwachs | 5% |
| | Bienenwachs | 10% |
| Phase B | Octyldodecanol | 30% |
| | Cocoglycerides | 10% |
| | Butyrospermum Parkii Butter | 5% |
| | UV-Filter | Ad 100 |
| Phase C | Ricinus Communis Seed Oil | 20% |
| | Pigment | Ad 100 |
| Phase D | Aroma | Ad 100 |

### Bulkherstellung

Phase A: Wachse aufschmelzen
Phase B: Zu Phase A geben
Phase C: Farbe in Rizinusöl dispergieren, zu Phase A+B geben
Erhitzen auf mind. 85°C, sodass alles gelöst/geschmolzen ist
Phase D dazugeben

### Abfüllung

- Masse (Bulk) auf Gießtemperatur (70°C) kühlen. Der Erstarrungspunkt der Masse liegt bei ca. 60°C
- Mithilfe der Abfüllmaschine in den Gießkranz füllen. Gießkranztemperatur: 12°C.
- Stifte mechanisch entformen.

Vor der (erneuten) Befüllung wird der Gießkranz auf 17°C erwärmt.

## Patentansprüche

1. Lippenpflegeprodukt in Form eines Lippenstiftes, enthaltend eine Wachsmischung aus
a) Rizinuswachs (INCI: Hydrogenated Castor Oil),
b) Sonnenblumenwachs (INCI: Helianthus Annuus Seed Cera) und
c) Bienenwachs (INCI: Cera Alba),
wobei die Zubereitung frei ist von Mineralöl, Paraffinwachs, mikrokristallinem Wachs, Schellackwachs und Polyethylenwachsen,
**dadurch gekennzeichnet, dass** die Zubereitung das Sonnenblumenwachs und das Rizinuswachs in einem Gewichtsverhältnis von 1:1 bis 4:1 enthält.

2. Lippenpflegeprodukt nach Anspruch 1, **dadurch gekennzeichnet, dass** die Zubereitung das Rizinuswachs in einer Konzentration von 2 bis 10 Gewichts-%, bezogen auf das Gesamtgewicht der Zubereitung, enthält.

3. Lippenpflegeprodukt nach einem der vorhergehenden Ansprüche, **dadurch gekennzeichnet, dass** die Zubereitung das Sonnenblumenwachs in einer Konzentration von 2 bis 10 Gewichts-%, bezogen auf das Gesamtgewicht der Zubereitung, enthält.

4. Lippenpflegeprodukt nach einem der vorhergehenden Ansprüche, **dadurch gekennzeichnet, dass** die Zubereitung das Bienenwachs in einer Konzentration von 5 bis 20 Gewichts-%, bezogen auf das Gesamtgewicht der Zubereitung, enthält.

5. Lippenpflegeprodukt nach einem der vorhergehenden Ansprüche, **dadurch gekennzeichnet, dass** die Zubereitung 30 bis 60 Gewichts-% an Ölen gewählt aus der Gruppe der Verbindungen Octyldodecanol, Rizinusöl (INCI: Ricinus Communis Seed Oil), Kokosglyceriden (INCI: Coco Glycerides), Jojobaöl (INCI: Simmondsia Chinensis Seed Oil).

6. Lippenpflegeprodukt nach einem der vorhergehenden Ansprüche, **dadurch gekennzeichnet, dass** die Zubereitung Cetylpalmitat, Sheabutter und/oder Cetearylalkohol enthält.

7. Lippenpflegeprodukt nach einem der vorhergehenden Ansprüche, **dadurch gekennzeichnet, dass** die Zubereitung Butylhydroxytoluol (BHT) enthält.

8. Lippenpflegeprodukt nach einem der vorhergehenden Ansprüche, **dadurch gekennzeichnet, dass** die Zubereitung UV-Filter, Farbstoffe, Parfümstoffe und/oder Aromastoffe enthält.

9. Lippenpflegeprodukt nach Anspruch 9, **dadurch gekennzeichnet, dass** der oder die Farbstoffe gewählt werden aus den Verbindungen mit den Color-Indices CI77891, CI 77492, CI 15850.

10. Lippenpflegeprodukt nach einem der Ansprüche 9 oder 10, **dadurch gekennzeichnet, dass** die UV-Filter gewählt werden aus der Gruppe der Verbindungen Octocrylen, Homosalat, Ethylhexylsalicylat, Ethylhexylmethoxycinnamat und Butyl Methoxydibenzoylmethan.

11. Lippenpflegeprodukt nach einem der vorhergehenden Ansprüche, **dadurch gekennzeichnet, dass** die Zubereitung Bis-Diglyceryl Polyacyladipate-2 enthält.

12. Verfahren zur Herstellung eines Lippenstiftes nach einem der vorhergehenden Ansprüche, **dadurch gekennzeichnet, dass** zuerst die Rezepturbestandteile in einem Heizkessel aufgeschmolzen und homogen vermischt werden, die Zubereitung anschließend bei einer Temperatur von 5-15 °C über ihrem Erstarrungspunkt in einen Gießkranz gegossen wird, dann mit einer Kühltemperatur von +6 °C bis +25 °C abgekühlt wird und die erstarrte lippenstiftförmige Masse aus dem Gießkranz herausgedrückt und in einen Lippenstiftbehälter eingebracht wird, wobei die Zubereitung das Sonnenblumenwachs und das Rizinuswachs in einem Gewichtsverhältnis von 1:1 bis 4:1 enthält.

13. Lippenstift hergestellt nach einem Verfahren nach Anspruch 12.

## Claims

1. Lip care product in the form of a lipstick, comprising a wax mixture consisting of
a) castor wax (INCI: Hydrogenated Castor Oil),
b) sunflower wax (INCI: Helianthus Annuus Seed Cera) and
c) beeswax (INCI: Cera Alba),
wherein the preparation is free of mineral oil, paraffin wax, microcrystalline wax, shellac wax and polyethylene waxes,
**characterized in that** the preparation comprises the sunflower wax and the castor wax in a weight ratio of 1:1 to 4:1.

2. Lip care product according to Claim 1, **characterized in that** the preparation comprises the castor wax at a concentration of 2% to 10% by weight, based on the total weight of the preparation.

3. Lip care product according to either of the preceding claims, **characterized in that** the preparation comprises the sunflower wax at a concentration of 2% to 10% by weight, based on the total weight of the preparation.

4. Lip care product according to any of the preceding claims, **characterized in that** the preparation comprises the beeswax at a concentration of 5% to 20% by weight, based on the total weight of the preparation.

5. Lip care product according to any of the preceding claims, **characterized in that** the preparation 30% to 60% by weight of oils selected from the following group of compounds: octyldodecanol, castor oil (INCI: Ricinus Communis Seed Oil), cocoglycerides (INCI: Cocoglycerides), jojoba oil (INCI: Simmondsia Chinensis Seed Oil).

6. Lip care product according to any of the preceding claims, **characterized in that** the preparation comprises cetyl palmitate, shea butter and/or cetearyl alcohol.

7. Lip care product according to any of the preceding claims, **characterized in that** the preparation comprises butylated hydroxytoluene (BHT).

8. Lip care product according to any of the preceding claims, **characterized in that** the preparation comprises UV filters, colourants, perfumes and/or aroma substances.

9. Lip care product according to Claim 9, **characterized in that** the colourant(s) is (are) selected from compounds with the colour indices CI77891, CI 77492, CI 15850.

10. Lip care product according to either of Claims 9 and 10, **characterized in that** the UV filters are selected from the following group of compounds: octocrylene, homosalate, ethylhexyl salicylate, ethylhexyl methoxycinnamate and butyl methoxydibenzoylmethane.

11. Lip care product according to any of the preceding claims, **characterized in that** the preparation comprises Bis-Diglyceryl Polyacyladipate-2.

12. Process for producing a lipstick according to any of the preceding claims, **characterized in that** the formulation constituents are firstly melted and homogeneously mixed in a boiler, the preparation is subsequently poured into a casting annulus at a temperature of 5-15°C above its solidification point, then is cooled at a cooling temperature of +6°C to +25°C and the solidified lipstick-shaped compound is expelled from the casting annulus and inserted into a lipstick container, wherein the preparation comprises the sunflower wax and the castor wax in a weight ratio of 1:1 to 4:1.

13. Lipstick produced by a process according to Claim 12.

## Revendications

1. Produit de soin des lèvres sous forme d'un rouge à lèvres contenant un mélange de cires constitué par
a) de la cire de ricin (INCI : Hydrogenated Castor Oil),
b) de la cire de tournesol (INCI : Helianthus Annuus Seed Cera) et
c) de la cire d'abeille (INCI : Cera Alba)
la préparation étant exempte d'huile minérale, de cire de paraffine, de cire microcristalline, de cire de gomme laque et de cires de polyéthylène,
**caractérisé en ce que** la préparation contient la cire de tournesol et la cire de ricin dans un rapport pondéral de 1:1 à 4:1.

2. Produit de soin des lèvres selon la revendication 1, **caractérisé en ce que** la préparation contient la cire de ricin en une concentration de 2 à 10% en poids, par rapport au poids total de la préparation.

3. Produit de soin des lèvres selon l'une des revendications précédentes, **caractérisé en ce que** la préparation contient la cire de tournesol en une concentration de 2 à 10% en poids, par rapport au poids total de la préparation.

4. Produit de soin des lèvres selon l'une des revendications précédentes, **caractérisé en ce que** la préparation contient la cire d'abeille en une concentration de 5 à 20% en poids, par rapport au poids total de la préparation.

5. Produit de soin des lèvres selon l'une des revendications précédentes, **caractérisé en ce que** la préparation 30 à 60% en poids d'huiles choisies dans le groupe des composés octyldodécanol, huile de ricin (INCI : Ricinus Communis Seed Oil), glycérides de coco (INCI : Coco glycerides), huile de jojoba (INCI : Simmondsia Chinensis Seed Oil).

6. Produit de soin des lèvres selon l'une des revendications précédentes, **caractérisé en ce que** la préparation contient du palmitate de cétyle, du beurre de karité et/ou de l'alcool cétéarylique.

7. Produit de soin des lèvres selon l'une des revendications précédentes, **caractérisé en ce que** la préparation contient du butylhydroxytoluène (BHT).

8. Produit de soin des lèvres selon l'une des revendications précédentes, **caractérisé en ce que** la préparation contient des filtres UV, des colorants, des parfums et/ou des arômes.

9. Produit de soin des lèvres selon la revendication 9, **caractérisé en ce que** le ou les colorants sont choisis parmi les composés présentant les indices de couleur CI77891, **CI** 77492, CI 15850.

10. Produit de soin des lèvres selon l'une des revendications 9 ou 10, **caractérisé en ce que** les filtres UV sont choisis dans le groupe des composés octocrylène, homosalate, salicylate d'éthylhexyle, méthoxycinnamate d'éthylhexyle et butylméthoxydibenzoylméthane.

11. Produit de soin des lèvres selon l'une des revendications précédentes, **caractérisé en ce que** la préparation contient du polyacyladipate-2 de bis-diglycéryle.

12. Procédé de fabrication d'un rouge à lèvres selon l'une des revendications précédentes, **caractérisé en ce que** les constituants de formulation sont d'abord fondus dans une cuve chauffée et mélangés de manière homogène, puis la préparation est coulée dans un anneau de coulée à une température de 5-15°C au-dessus de son point de solidification, puis refroidie à une température de refroidissement de +6°C à +25°C et la masse solidifiée en forme de rouge à lèvres est pressée hors de l'anneau de coulée et introduite dans un récipient à rouge à lèvres, la préparation contenant la cire de tournesol et la cire de ricin dans un rapport pondéral de 1:1 à 4:1.

13. Rouge à lèvres fabriqué selon un procédé selon la revendication 12.
